# FASCICULE DE BREVET EUROPEEN

(11) **EP 0 300 844 B1**
(45) Date de publication et mention de la délivrance du brevet: **01.09.1993**
(21) Numéro de dépôt: 88401474.7
(22) Date de dépôt: 15.06.1988
(51) Int. Cl.: C07C 69/587, C07C 67/02, C07C 67/56, A23L 1/30

(54) **Nouveaux glycérides d'acide gras insaturé et leurs procédés d'obtention**
Ungesättigte Fettsäureglyceride und ihre Herstellungsverfahren
Glycerides of unsaturated fetty acids and their methods of preparation

(30) Priorité: 29.06.1987 FR 8709114
(43) Date de publication de la demande: 25.01.1989
(73) Titulaire: Azar, Roger F., F-75007 Paris (FR); Grinda, Jean-Robert, F-75016 Paris (FR)
(72) Inventeur: Azar, Roger F., 75007 Paris (FR); Grinda, Jean Robert, 75006 Paris (FR); Mac Farlan, Neal Hawaiian Agronomic Biotech. Ltd., Hull HU 2 OAD (GB)
(74) Mandataire: Burtin, Jean-François (FR)

(56) Documents cités:
- EP-A- 0 092 085
- EP-A- 0 224 799
- DE-A- 2 749 492
- FR-A- 2 515 174
- FR-A- 2 588 187

## Description

La présente invention se rapporte à de nouveaux glycérides d'acide gras poly-insaturé obtenus à l'état pur et à leurs procédés d'obtention.

Elle a plus particulièrement pour objet les nouveaux glycérides de l'acide (cis, cis, cis) octadécatriénoïque (6,9,12) ou acide gamma-linolénique chimiquement et stériquement purs.

Elle a spécifiquement pour objet, les glycérides de formule générale I : 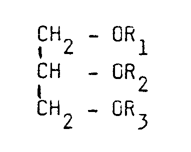
dans laquelle
R₂ est le reste acyle de l'acide (cis,cis,cis)octadécatriénoïque (6,9,12) et
R₁ et R₃, semblables ou différents, sont de l'hydrogène, un reste acétyle ou le reste acyle de l'acide cis,cis,cis-octadécatriénoïque (6,9,12).

Le procédé selon l'invention permet donc d'obtenir :
- le triglycéride pur de l'acide cis,cis,cis-octadécatriénoïque (6,9,12) ou trigamma-linolénine qui est un composé nouveau
- les diglycérides 1,3- ou 2,3- de l'acide cis,cis,cis-octadécatriénoïque (6,9,12), ce dernier étant prédominant car l'estérification de l'alcool secondaire du glycérol intervient en premier lieu.
- les glycérides mixtes de l'acide gamma-linolénique et de l'acide acétique résultant d'une inter-estérification partielle de la triacétine par l'acide gamma-linolénique.

Tous ces composés stériquement purs sont des composés nouveaux.

On sait le rôle joué par l'acide gamma-linolénique en diététique et en pharmacologie. Il fait partie des acides gras essentiels avec l'acide α-linolénique et l'acide linoléique. Ce sont des substances qui se comportent comme des vitamines car l'organisme n'est pas en mesure d'en faire la synthèse et, de ce fait, ils doivent être fournis par l'alimentation.

En outre, l'organisme sait transformer l'acide gamma-linolénique en son homologue supérieur ayant deux atomes de carbone supplémentaire, l'acide dihomogamma-linolénique ou acide éicosatriénoïque-8,11,14. Cette voie métabolique joue un rôle essentiel. En effet, cette transformation n'est pas réversible chez l'homme et conduit par des étapes successives sous l'influence alternée de désaturases spécifiques et de réaction d'homologation à l'acide arachidonique (acide 5,8,11,14 -éicosatétraénoïque). Cette voie métabolique est bien connue maintenant et apporte la preuve que l'acide gamma-linolénique et les autres acides gras insaturés agissent en tant que précurseurs de la synthèse des prostaglandines de la série 1 qui dérivent de l'acide dihomo gamma-linolénique (DGLA) et des prostaglandines de la série 2 qui sont formés au stade suivant à partir de l'acide arachidonique. Enfin, on a constaté plus récemment que les acides gras en C₂₂ à 4 doubles liaisons formés par métabolisation de l'acide arachidonique conduisent à des homoprostaglandines de la série 2 (voir en particulier, la demande de brevet européen 211 502).

L'acide gamma-linolénique est donc l'élément clef de cette voie métabolique car il est transformé complètement et rapidement en acide dihomogamma-linolénique. En pratique, administrer de l'acide gamma-linolénique revient à administrer de l'acide dihomogamma-linolénique.

Les sources naturelles d'acide gamma-linolénique sont peu nombreuses. On peut citer, à cet égard, l'huile d'onagre, l'huile de pépins de cassis ou de framboisier et l'huile de Bourrache. Dans la plupart des huiles, on constate la présence prépondérante d'acide linoléique et d'acide α-linolénique. Seule l'huile de Bourrache fournit une quantité importante d'acide gamma-linolénique. Dans la nature, l'acide gamma-linolénique, comme les autres acides gras insaturés, existent sous forme de glycérides et en particulier sous forme de glycérides mixtes, dans lesquels l'acide gamma-linolénique est de préférence fixé sur la fonction alcool en 2 du glycérol. Les autres fonctions alcool sont estérifiées par un ou deux autres acides gras du même type.

Les glycérides d'acides gras insaturés constituent la forme d'administration la plus efficace car ils sont utilisés dans l'organisme alors que l'acide lui-même est très peu résorbé.

C'est pourquoi, en raison de l'importance de l'acide gamma-linolénique dans cette chaîne métabolique, il était important de disposer de glycérides d'acide gamma-linolénique purs chimiquement et de configuration spatiale cis,cis,cis absolument définie.

On connaissait déjà par la littérature, un produit dénommé trilinolénine obtenu notamment à partir de l'huile de lin ou par hémisynthèse à partir d'acide linolénique et de glycérol. En fait, ces produits se sont avérés être des esters plus ou moins mixtes d'acide α-linolénique et d'acide γ-linolénique. Les constantes physiques très diverses mentionnées dans la littérature indiquent que les produits obtenus sont des mélanges de glycérides ou des mélanges de diastéréo-isomères. On a également obtenu un mélange riche en triglycéride de l'acide gamma-linolénique par chauffage de l'acide impur avec du glycérol (voir demande européenne 178.442) sans qu'il soit possible de déterminer si le chauffage en milieu acide n'a pas dégradé également la chaine glycéryle.

Les différentes données de la littérature ont toujours eu pour objectif de préparer de l'acide γ-linolénique pur, c'est-à-dire séparé de l'acide α-linolénique, avec lequel il est mélangé dans un grand nombre d'huiles. Le brevet européenn 092.085, cité dans le rapport de recherche, montre que l'huile de pépins de cassis contient 15 à 19% d'acide γ-linolénique (3ω6) et 12 à 14% d'acide α-linolénique (3ω3). La séparation de ces deux acides gras non saturés est difficile et le brevet européen 178.442 fournit un mode d'enrichissement en acide γ-linolénique de l'huile de cassis par élimination sélective de l'acide α-linolénique et de l'acide stéaridonique (homologue à quatre double liaisons). On obtient, grâce à ce procédé, un acide γ-linolénique pur à 96% et contaminé par 4% d'autres acides gras.

Quand on part de cet acide γ-linolénique presque pur chimiquement, on peut obtenir un mélange de triglycérides (exemple 5 du brevet européen 178.442) dont le degré de pureté chimique est inconnu.

On est, par ailleurs, surpris à la lecture de la littérature de constater l'extrême diversité des constantes physiques fournies pour le composé dénommé γ-trilinolénine, c'est-à-dire le triglycéride de l'acide γ-linolénique et le degré de pureté chimique du produit obtenu peut être réellement mis en cause.

D'autre part, les synthèses chimiques des acides gras polyinsaturés datent d'une époque (1950-1960) où les problèmes de stéréochimie étaient difficiles à résoudre et où les méthodes analytiques manquaient de sensibilité.

C'est ainsi que la publication David R.HOWTON et Coll (J. of Am. Chem. Soc. 76 (1954) 4971) indique que la substance employée dans le passé contient environ 15% d'un ou deux acides isomériques cis-trans. Ceci est confirmé dans la partie expérimentale (page 4974).

C'est la raison pour laquelle en partant d'un acide γ-linolénique pur, dans les meilleurs conditions, les auteurs ont pu préparer un ester méthylique d'une pureté souvent améliorée, car il est possible de le purifier par passage sur résine argentique ou mercurielle ou par distillation fractionnée.

Malgré toutes ces études destinées principalement à former un composé marqué au ¹⁴C pour faire des études biologiques, la recherche d'un acide γ-linolénique pur stéréochimiquement était restée purement spéculative.

Or, il se trouve que les études plus récentes ont montré que l'acide γ-linolénique comme d'autres acides gras polyinsaturés ne sont actifs qu'après conversion en homologues supérieurs (acide dihomo γ-linolénique ou acide stéaridonique) et que cette métabolisation s'effectue essentiellement par voie enzymatique sous l'intervention d'une désaturase (Δ5-désaturase ou Δ6-désaturase). L'étude par modèle des différents stéreoisomères de l'acide γ-linolénique a montré que c'était l'acide cis/cis/cis qui avait la conformation la plus proche de celle du récepteur enzymatique de la desaturase et, par conséquent, la recherche d'un acide entièrement cis devait conduire à un produit dont l'activité serait bien supérieure à celle des acides γ-linoléniques de la littérature même purs à 100% chimiquement.

D'après les analyses effectuées, le triglycéride de l'acide cis-cis-cis γ-linolénique, objet de la présente demande de brevet, ne présente, au pire, que des indications de traces de liaisons trans (selon les constatations des analystes) et on peut considérer que c'est la première fois que l'on obtient effectivement, en l'isolant, un triglycéride de cet acide stéréochimiquement pur.

La présente invention a essentiellement pour objet le triglycéride de l'acide gamma-linolénique pur. Celui-ci est obtenu à partir d'un gamma-linolénate d'alcoyle pur, préparé par alcoolyse rapide d'une fraction d'une huile riche en acide gamma-linolénique, comme par exemple, l'huile de Bourrache.

Au cours de la préparation du triglycéride de l'acide gamma-linolénique, il se forme des produits secondaires, à savoir le mono-glycéride de l'acide gamma-linolénique et les diglycérides de l'acide gamma-linolénique, ainsi que des glycérides mono- ou diacétylés.

Ces glycérides purs sont obtenus par un procédé qui consiste à faire réagir un ester d'alcoyle inférieur de l'acide gamma-linolénique pur avec la triacétine, en présence d'un agent basique pour obtenir essentiellement le triglycéride de l'acide gamma-linolénique et une petite quantité de mono- et de diglycérides de l'acide gamma-linolénique.

La réaction de synthèse selon l'invention peut donc s'écrire : 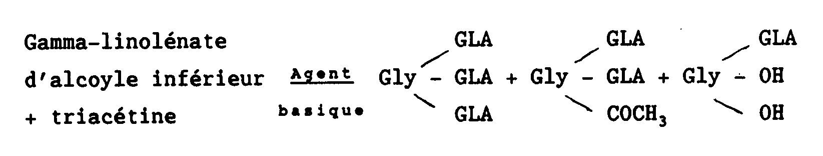
La triacétine a pour formule :

CH₂OCOCH₃

CHOCOCH₃

CH₂OCOCH₃

Elle est capable d'échanger un ou plusieurs groupes acétyle en milieu basique avec un autre groupe acyle comme le radical octadécatriénoyle. Dans cette réaction l'agent basique est de préférence un alcoolate de métal alcalin code le méthanolate de sodium ou le lithium, l'éthanolate de sodium, le terbutylate de potassium ou le teramylate de potassium. L'agent basique est utilisé en quantité quasiment catalytique.

La réaction d'inter-estérification est effectuée à une température et/ou sous un vide tels que l'acétate d'alcoyle inférieur formé distille au fur et à mesure de sa formation. Cette séparation assure donc un déplacement constant de la réaction qui est finalement pratiquement totale. La formation de glycéride mixte reste faible.

De préférence, on utilise l'ester méthylique de l'acide (cis,cis,cis) octadécatriénoique (6,9,12) comme matière première et l'acétate de méthyle formé distille vers 65°. De cette façon, la réaction d'interestérification peut être conduite à température relativement basse.

On peut néanmoins utiliser d'autres esters de l'acide (cis,cis,cis) octadécatriénoique (6,9,12) comme l'ester éthylique, isopropylique ou butylique. Les conditions opératoires (température, pression) devront être ajustées de façon à ce que l'acétate d'alcoyle, correspondant qui se forme, soit distillé au fur et à mesure de sa formation.

Ce mélange est séparé sur une colonne de gel de silice qui retient le mono- et les diglycérides et fournit le triglycéride d'acide gamma-linolénique contaminé seulement par une petite quantité d'ester d'alcoyle de départ et d'un triglycéride mixte formé de deux restes acyles d'acide gamma-linolénique et d'un reste acétyle.

Le triglycéride pur d'acide gamma-linolénique est finalement obtenu par une méthode physique qui permet de séparer le triglycéride des esters d'alcoyle. La chromatographie en phase liquide en phase inverse est une méthode appropriée qui permet cette séparation dans les conditions optimales.

La chromatographie en phase liquide en phase inverse permet soit d'effectuer une séparation à l'échelle analytique, soit d'effectuer la séparation des deux constituants à l'échelle préparative. On utilise à cette fin, une colonne chargée de Bondapak C18.

L'ester d'alcoyle de l'acide gamma-linolénique pur (tout cis,cis,cis) est obtenu à partir d'une huile riche en acide gamma-linolénique comme l'huile de Bourrache, par une réaction d'alcoolyse ménagée qui consiste en ce que l'on met l'huile de Bourrache avec une solution de potasse dans l'alcanol inférieur convenable pendant un temps très court, inférieur à une minute. Dans ces conditions, l'huile de Bourrache ne subit qu'une saponification en acide gras très limitée et l'essentiel des glycérides présents est transformé en ester d'alcoyle inférieur.

L'ester d'alcoyle inférieur est lavé par des mélanges de cyclohexane et d'acétonitrile à concentrations croissantes pour éliminer les acides gras puis à l'eau et enfin purifié par chromatographie liquide haute performance (HPLC). On recueille ainsi un gamma-linolénate d'alcoyle (cis,cis,cis) absolument pur. En particulier, lorsqu'on utilise l'alcoolyse par le méthanol, on obtient le gamma-linolénate de méthyle (cis,cis,cis) absolument pur et dont la stéréochimie est prouvée par dichroisme circulaire et par le spectre de RMN (spectre du proton et spectre du BC).

Le gamma-linolénate de méthyle (cis,cis,cis) est un produit nouveau qui n'avait jamais été obtenu à l'état pur.

Il est possible également de purifier le mélange obtenu par alcoolyse de l'huile de Bourrache par chromatographie sur colonne chargée d'une résine polyvinylsulfonique sous forme de dérivé métallique. On sépare ainsi les acides gras en fonction de leur degré d'insaturation.

Les exemples suivants illustrent l'invention :

### EXEMPLE I

- Stade A :: 20 g d'ester méthylique pur d'acide gamma-linolénique et 0,44 g de méthylate de sodium sec sont mélangés dans une fiole conique et chauffés à 65°C sous vide poussé. On ajoute alors 4,44 g de triacétine goutte à goutte en une heure. Après l'achèvement de l'addition, le chauffage sous vide est poursuivi pendant un heure. L'acétate de méthyle formé au cours de la réaction est éliminé par distillation au fur et à mesure de sa formation. Le mélange réactionnel contient de 75 à 80% de triglycéride, 14% environ d'ester méthylique résiduel et 6 à 10% de mono- et de di-glycérides.
- Stade B :: Après refroidissement du mélange réactionnel, on filtre pour éliminer les produits basiques résultant de la décomposition du méthylate de sodium. On passe ensuite le filtrat sur une colonne de gel de silice de 20,3 x 5 cm (qualité chromatographique) en utilisant du n-hexane comme solvant. On recueille des éluats successifs renfermant du gamma-linolénate de méthyle qui n'a pas réagi, puis des triglycérides. Par chromatographie en phase gazeuse (GLC) de cette fraction (appelée fraction 1) qui pèse 10,7 g, on détecte la présence :
- d'un peu de gamma-linolénate de méthyle
- de triglycéride contenant 2 moles d'acide gamma-linolénique et un radical acétyle
   et
- essentiellement de la trigamma-linolénine pure.

Par élution de la colonne de gel de silice par de l'acétone, on obtient le monoglycéride de l'acide gamma-linolénique et les diglycérides de l'acide gamma-linolénique.

Le rendement en cette fraction (fraction 2) est de 6,2 g. Au total, au départ de 20 g de gamma-linolénate de méthyle, on obtient 16,9 g de dérivés glycériques et on récupère une certaine quantité de gamma-linolénate de méthyle.

La fraction 1 est soumise à une séparation par chromatographie préparative en phase liquide (HPLC) en phase inverse sur colonne de Bondapak C18. On sépare ainsi le triglycéride pur de l'acide cis,cis,cis gamma-linolénique (6,9,12).

Le spectre UV du Triglycéride a été déterminé dans le chloroforme aux concentrations de 40 mg de Triglycéride dans 10 ml de chloroforme (0.4%) et de 40 mg dans 20 ml de chloroforme (0.2%).

Les caractéristiques suivantes ont été obtenues :

| C | 0.2% | 0.4% |
|---|---|---|
| λmax | 248 nm | 250.5 nm |
| Absorption | 1,9341 | 2,7576 |

La pureté du Triglycéride a également été déterminée par spectromètrie IR. Le spectre IR montre essentiellement une absorption à 3.000 cm⁻¹ caractéristique de la double liaison et à 1570 cm⁻¹ caractéristique de la bande carbonyle.

Le spectre RMN du Carbone ¹³C montre deux massifs de pics l'un vers 130 et l'autre plus étendu entre 22 et 34 ppm.

Le spectre RMN du proton, montre des pics caractéristiques à 2,8-2,3-2,0 ppm et un massif important à 1,3 ppm.

Le triglycéride de l'acide (cis,cis,cis) octadécatriénoique (6,9,12) ou gamma-linolénine peut servir comme médicament en thérapeutique cardio-vasculaire ou comme produit diététique pour assurer à l'alimentation une teneur appropriée en acides gras poly-insaturés.

Le di, le triglycéride ainsi que le diglycéride O-acétylé de l'acide (cis,cis,cis) octadécatriénoique peuvent encore trouver un emploi dans des préparations cosmétologiques ou cosméto-dermatologiques.

Le mono- ou les di-glycérides de l'acide cis,cis,cis-octadécatriénoique (6,9,12) trouvent un emploi en diététique comme apport d'acides gras poly-insaturés essentiels.

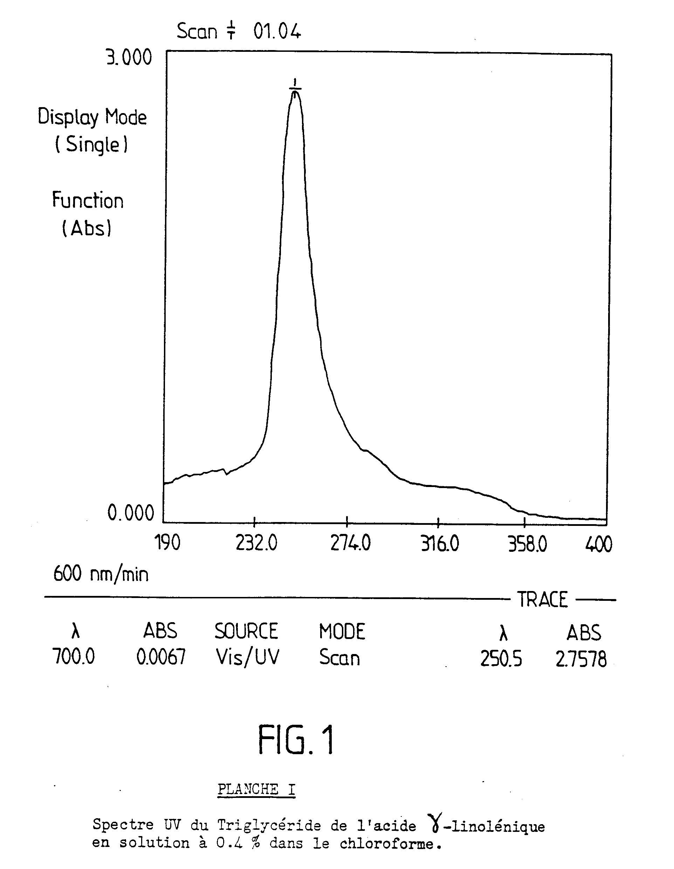

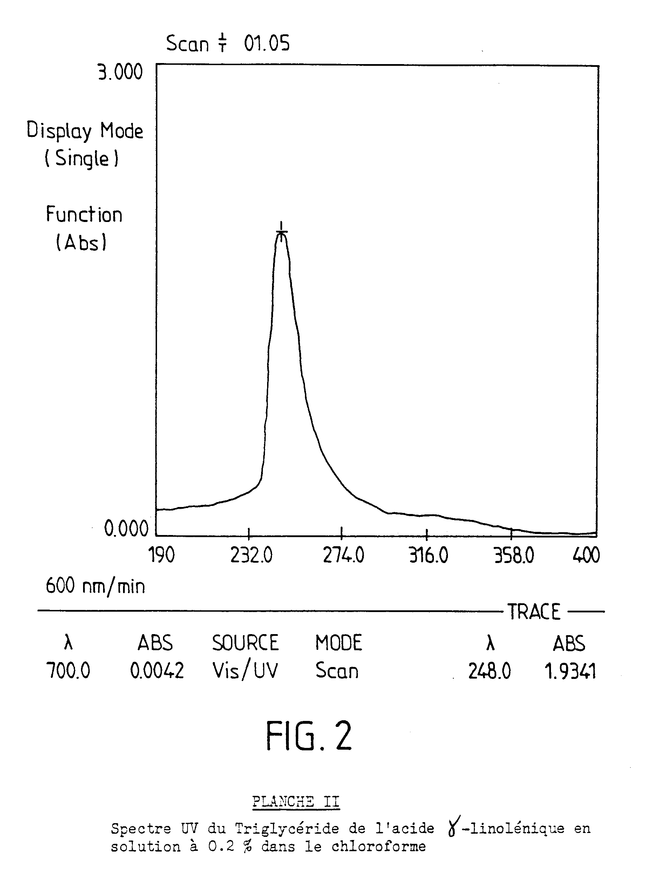

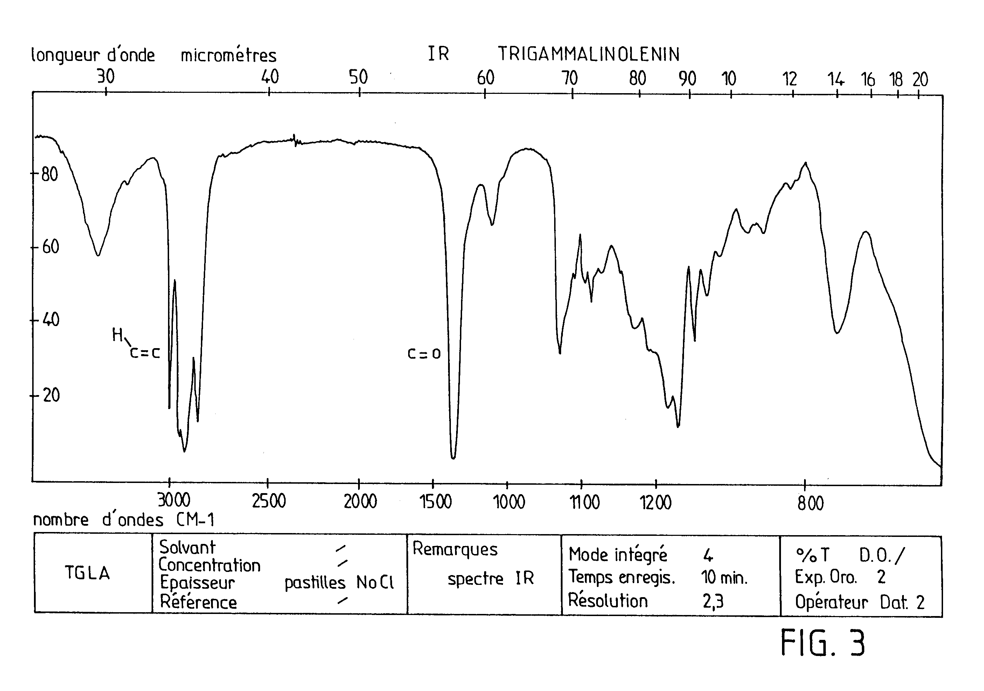

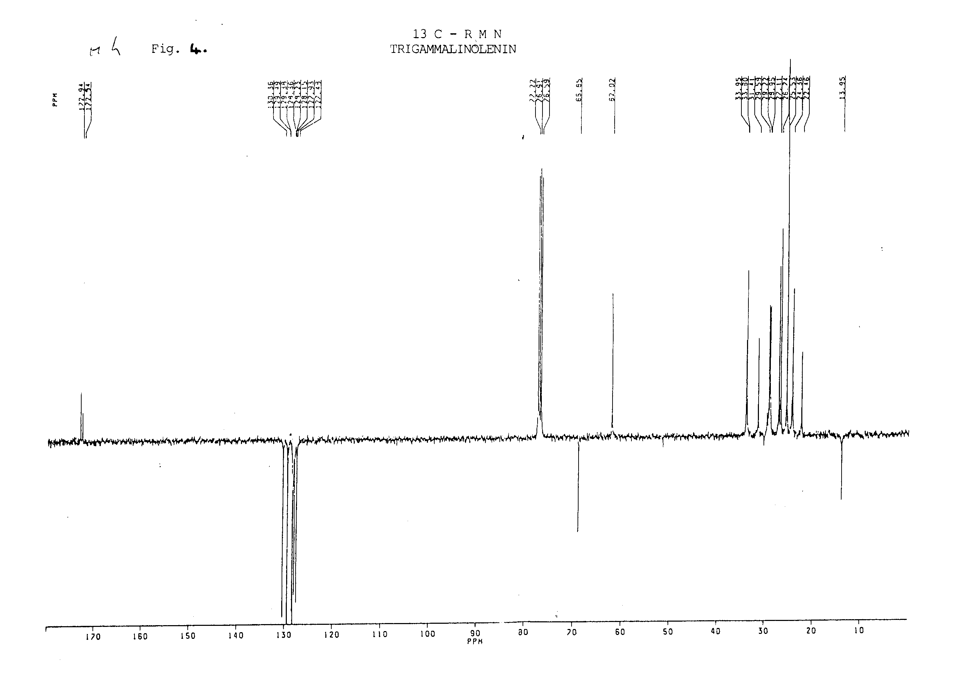

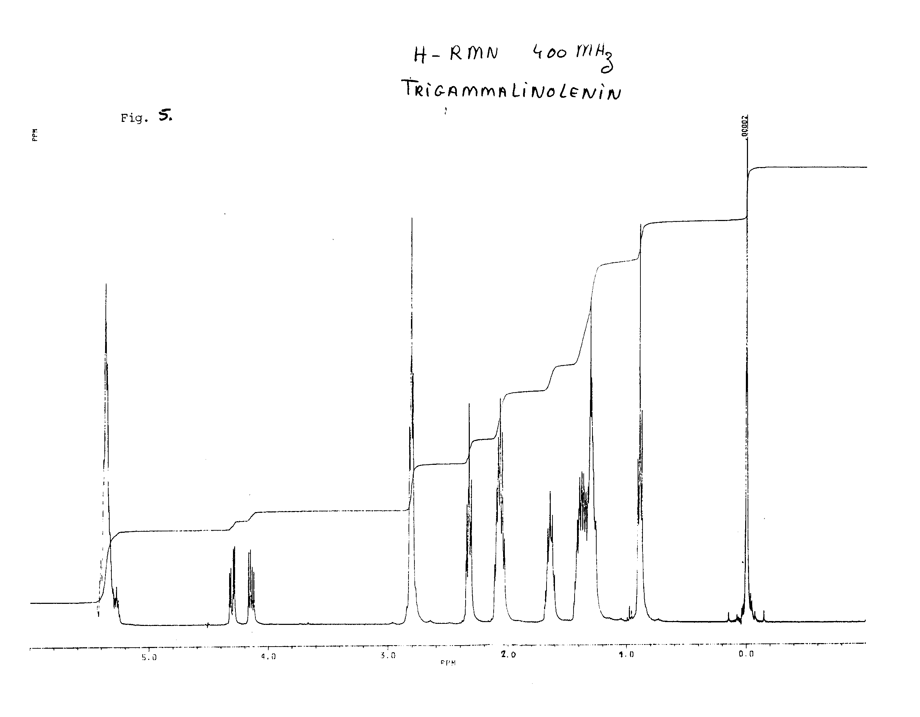

## Revendications (Revendications pour l'(les) Etat(s) contractant(s) suivant(s): AT, BE, CH, DE, FR, GB, IT, LI, LU, NL, SE)

1. Les glycérides de l'acide cis-cis-cis octadecatriénoique (6,9,12) sous forme pratiquement pure d'un point de vue stéréochimique, répondant à la formule générale I 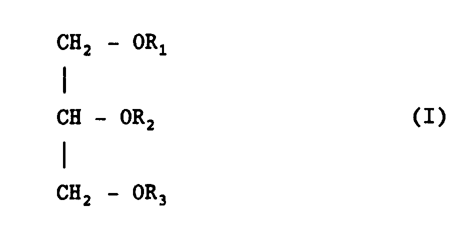 dans laquelle
R₂ est le reste acyle de l'acide (cis,cis,cis) octadécatriénoique (6,9,12),
R₁ et R₃, semblables ou différents, sont de l'hydrogène, un reste acétyle ou le reste acyle de l'acide cis,cis,cis-octadécatriénoique (6,9,12),
isolément ou en mélange.

2. Le triglycéride de l'acide cis,cis,cis-octadécatriénoique (6,9,12)

3. Le triglycéride mixte ayant deux restes acyle de l'acide cis-cis-cis octadécatriénoique (6,9,12) stéréochimiquement pur et un reste acétyle, isolément ou en mélange, avec son isomère de position.

4. Le monoglycéride de l'acide (cis,cis,cis) octadécatriénoique (6,9,12).

5. Les glycérides-2,3 et les diglycérides-1,3 de l'acide (cis,cis,cis) octadécatriénoique (6,9,12), isolément ou en mélanges avec leur isomère de position.

6. Un procédé d'obtention des glycérides de formule générale I selon l'une des revendications 1 à 5, dans lequel on fait réagir un ester d'alcoyle inférieur de l'acide (cis,cis,cis) octadécatriénoique (6,9,12) avec la triacétine, en présence d'un catalyseur comme un agent basique à une température et sous une pression où l'acétate d'alcoyle inférieur formé distille, sépare les triglycérides obtenus du mélange de mono- et de diglycérides puis fractionne par des moyens physiques le mélange de triglycérides pour obtenir séparement le triglycéride de l'acide cis,cis,cis-octadécatriénoique (6,9,12) et le diglycéride de l'acide cis,cis,cis-octadéca- triénoique (6,9,12) O-acétyle.

7. Un procédé selon la revendication 6°, dans lequel l'agent basique est un alcoolate de métal alcalin.

8. Un procédé selon l'une des revendications 6 et 7°, dans lequel l'alcoolate de métal alcalin est le méthanolate de sodium.

9. Un procédé selon l'une des revendications 6 à 8°, dans lequel l'ester d'alcoyle inférieur de l'acide (cis,cis,cis) octadécatriénoique est un ester ayant de 1 à 4 atomes de carbone.

10. Un procédé selon l'une des revendications 6 à 9° dans lequel la séparation des triglycérides est effectuée par chromatographie sur colonne de gel de silice.

11. Un procédé selon l'une des revendications 6 à 10° dans lequel le mélange de triglycérides est fractionné par chromatographie haute performance en phase liquide en phase inverse.

12. Utilisation du triglycéride de l'acide (cis,cis,cis) octadécatriénoique (6,9,12) comme médicament et/ou dans des compositions diététiques.

## Revendications (Revendications pour l'(les) Etat(s) contractant(s) suivant(s): ES, GR)

1. Un procédé d'obtention des glycérides de l'acide cis-cis-cis octadecatrienoique (6,9,12) sous forme pratiquement pure d'un point de vue stéréochimique, répondant à la formule générale I 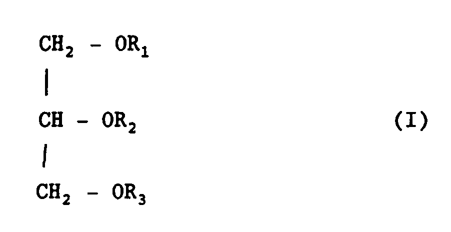 dans laquelle
R₂ est le reste acyle de l'acide (cis,cis,cis) octadecatriénoique (6,9,12)
R₁ et R₃, semblables ou différents, sont de l'hydrogène, un reste acétyle ou le reste acyle de l'acide cis,cis,cis-octadécatriénoique (6,9,12), isolement ou en mélange, dans lequel on fait réagir un ester d'alcoyle inférieur de l'acide (cis,cis,cis) octadécatriénoique (6,9,12) avec la triacétine, en présence d'un catalyseur comme un agent basique à une température et sous une pression où l'acétate d'alcoyle inférieur formé distille, sépare les triglycérides obtenus du mélange de mono- et de diglycérides puis fractionne par des moyens physiques le triglycéride de l'acide cis,cis,cis-octadécatriénoique (6,9,12) et le diglycéride de l'acide cis,cis,cis-octadécatriénoique (6,9,12) O-acétyle.

2. Un procédé selon la revendication 1° dans lequel l'agent basique est un alcoolate de métal alcalin.

3. Un procédé selon l'une des revendications 1 et 2°, dans lequel l'alcoolate de métal alcalin est le méthanolate de sodium.

4. Un procédé selon l'une des revendications 6 à 8, dans lequel lester d'alcoyle inférieur de l'acide (cis,cis,cis)octadécatriénoïque est un ester ayant de 1 à 4 atomes de carbone.

5. Un procédé selon l'une des revendications 6 à 9 dans lequel la séparation des triglycérides est effectuée par chromatographie sur colonne de gel de silice.

6. Un procédé selon l'une des revendications 6 à 10 dans lequel le mélange de triglycérides est fractionné par chromatographie haute performance en phase liquide en phase inverse.

7. Utilisation du triglycéride de l'acide (cis,cis,cis)octadécatriénoïque (6,9,12) dans des compositions diététiques.

## Claims (Claims for the following Contracting State(s): AT, BE, CH, DE, FR, GB, IT, LI, LU, NL, SE)

1. The glycerides of cis-cis-cis octadecatrienoic acid (6,9,12) in a practically pure form from a stereochemical point of view, having the general formula I : 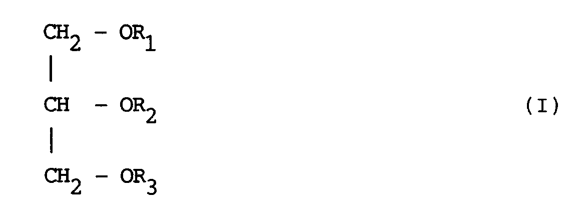 wherein
R₂ is the acyl residue of (cis,cis,cis) octadeca (6,9,12) Trienoic acid
R₁ and R₃, the same or different are a hydrogen, an acetyl residue or the acyl rest of cis,cis,cis-octadeca (6,9,12) Trienoic acid,
as a single compound or in a mixture.

2. The triglyceride of cis,cis,cis-octadeca (6,9,12) Trienoic acid.

3. The mixed triglyceride having two acyl residues from stereo chemically pure cis,cis,cis-octadeca (6,9,12) Trienoic acid and one acetyl rest,
as a single compound or in a mixture with its position isomer.

4. The monoglyceride of (cis,cis,cis) octadeca (6,9,12) Trienoic acid.

5. The 2,3-glycerides and the 1,3-glycerides of (cis,cis,cis) octadeca (6,9,12) Trienoic acid, as a single compound or in a mixture with their position isomer.

6. A process for producing the glycerides of formula I according to one of Claims 1° to 5° in which a lower alkyl ester of cis,cis,cis - octadeca (6,9,12) Trienoic acid is reacted with triacetin in the presence of a catalyst such as a basic agent at a temperature and under a pressure where the thus formed lower alkyl acetate distillates, the produced triglycerides are separated from the mixture of mono- and diglycerides then the mixture of triglycerides is fractionated by physical means to obtain separately the triglyceride of cis,cis,cis - octadeca (6,9,12) Trienoic acid and the diglyceride of O-acetyl cis,cis,cis - octadeca (6,9,12) Trienoic acid.

7. A process according to claim 6° wherein the basic agent is an alkali metal alcoholate.

8. A process according to one of the claims 6° and 7° wherein the alkali metal alcoholate is sodium methanolate.

9. A process according to one of the claims 6° to 8° wherein the lower alkyl ester of cis,cis,cis - octadeca (6,9,12) - Trienoic acid is an ester having from 1 to 4 carbon atoms.

10. A process according to one of the claims 6° to 9° wherein the separation of the triglycerides is performed by chromatography on a column of silica gel.

11. A process according to one of the claims 6° to 10° wherein the mixture of triglycerides is partitioned using high performance chromatography in a liquid phase in reverse phase.

12. Use of the triglyceride of cis,cis,cis - octadeca (6,9,12) - Trienoic acid as a drug and/or in dietetic compositions.

## Claims (Claims for the following Contracting State(s): ES, GR)

1. A process for producing the glycerides of cis-cis-cis octadecatrienoic acid (6,9,12) in a practically pure form from a stereochemical point of view, having the general formula I : 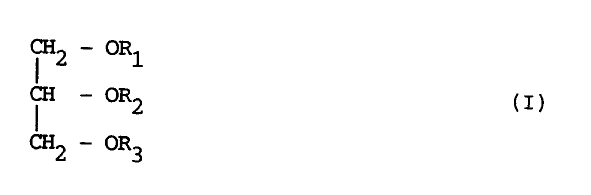 wherein
R₂ is the acyl residue of (cis,cis,cis)octadeca (6,9,12) Trienoic acid
R₁ and R₃ the same or different are a hydrogen, an acetyl residue or the acyl rest of cis,cis,cis octadeca (6,9,12) Trienoic acid as a single compound or in a mixture
in which a lower alkyl ester of cis,cis,cis - octadeca (6,9,12) Trienoic acid is reacted with triacetin in the presence of a catalyst such as a basic agent at a temperature and under a pressure where the thus formed lower alkyl acetate distillates, the produced triglycerides are separated from the mixture of mono- and diglycerides then the mixture of triglycerides is fractionated by physical means to obtain separately the triglyceride of cis,cis,cis - octadeca (6,9,12) Trienoic acid and the diglyceride of O-acetyl cis,cis,cis - octadeca (6,9,12) Trienoic acid.

2. A process according to the claim 1° wherein the basic agent is an alkali metal alcoholate.

3. A process according to one of the claims 1° and 2° wherein the alkali metal alcoholate is sodium methanolate.

4. A process according to one of the claims 1° and 2° wherein the lower alkyl ester of cis,cis,cis - octadeca (6,9,12) - Trienoic acid is an ester having from 1 to 4 carbon atoms.

5. A process according to one of the claims 1° to 4° wherein the separation of the triglycerides is performed using chromatography on a column of silica gel.

6. A process according to one of the claims 1° to 4° wherein the mixture of triglycerides is partitioned using high performance chromatography in a liquid phase in reverse phase.

7. Use of the triglyceride of cis,cis,cis - octadeca (6,9,12) - Trienoic acid in dietetic compositions whenever is obtained according to the process of the claim 1°.

## Patentansprüche (Patentansprüche für folgende(n) Vertragsstaat(en): AT, BE, CH, DE, FR, GB, IT, LI, LU, NL, SE)

1. Die Triglyzeride von cis,cis,cis Oktadekatrienoic Säure (6,9,12) in bezuglich der Stereochemie praktischen reinen Form der allgemeinen Formel I : 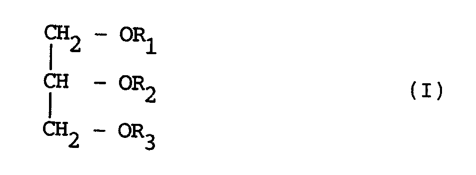 worin
R₂ der Acylrest von cis,cis,cis Oktadekatrienoic Säure (6,9,12) ist
R₁ und R₃, gleich oder verschiedene, Wasserstoff, ein Acetylrest oder der Acylrest von cis,cis,cis Oktadekatrienoic Säure (6,9,12) sind, allein oder in Gemisch.

2. Das Triglyzerid der cis,cis,cis Oktadekatrienoic Säure (6,9,12).

3. Das Mischtriglyzerid, das zwei Acylreste der stereochemisch reinen cis,cis,cis Oktadekatrienoic Säure (6,9,12) und ein Acetylrest enthält, allein oder in Gemisch mit sein Stellungsisomer.

4. Das Monoglyzerid der cis,cis,cis Oktadekatrienoic Säure (6,9,12).

5. Die Glyzeride-2,3 und die Glyzeride-1,3 der cis,cis,cis Oktadekatrienoic Säure (6,9,12), allein oder in Gemisch mit ihre Stellungsisomere.

6. Verfahren zur Herstellung von Glyzeriden der allgemeinen Formel I worin man ein niedrig alkyl Ester der cis,cis,cis Oktadekatrienoic Säure (6,9,12) mit Triazetin in Gegenwart eines Katalysators wie eine basischen Mittel, bei einer Temperatur und bei einer Druck wo der niedrig alkyl Azetate siedet, umsetzt, man die erhaltene Triglyzeride aus der Gemisch von Mono- und Diglyzeriden abtrennt, dann mittels physischen Mitteln den Gemisch aus Triglyzeriden fraktionniert, um das Triglyzerid von cis,cis,cis Oktadekatrienoic Säure (6,9,12) und das Diglyzerid von O-acetyl cis,cis,cis Oktadekatrienoic Säure separat zu gewinnen.

7. Verfahren nach Anspruch 6 worin das basische Mittel ein Alkalimetal Alkoholate ist.

8. Verfahren nach einer der Ansprüche 6 und 7 worin das Alkalimetal Alkoholate Sodium Methanolate ist.

9. Verfahren nach einer der Ansprüche 6 bis 8 worin das niedrig alkyl Ester von cis,cis,cis Oktadekatrienoic Säure ein Ester mit von 1 bis 4 Kohlenstoff Atomen ist.

10. Verfahren nach einer der Ansprüche 6 bis 9 worin die Abtrennung von der Triglyzeride durch chromatographie uber eine Säule von Silica Gel durchführt wird.

11. Verfahren nach einer der Ansprüche 6 bis 10 worin das Gemisch von Triglyzeriden mittels High Performance Liquid Chromatography in reverse phase, fraktionniert wird.

12. Verwendung von des Triglyzerid der (cis,cis,cis) Oktadekatrienoic Säure (6,9,12) als Arzneimittel und/oder in diätetischen Zubereitungen.

## Patentansprüche (Patentansprüche für folgende(n) Vertragsstaat(en): ES, GR)

1. Verfahren zur Herstellung von in, bezuglich Stereochemie praktisch reinen Form, Glyzeride von cis,cis,cis Oktadekatrienoic Säure (6,9,12), entsprechend der allgemeinen Formel I : 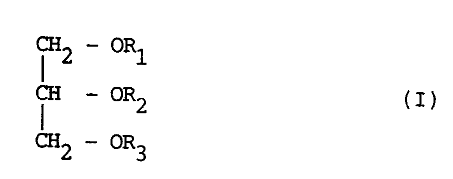 worin
R₂ der Acylrest von cis,cis,cis Oktadekatrienoic Säure (6,9,12) ist
R₁ und R₃, gleich oder verschiedene, Wasserstoff, Acetylrest oder der Acylrest von cis,cis,cis Oktadekatrienoic Säure (6,9,12) sind,
allein oder in Gemisch
worin man ein niedrigalkylester von (cis,cis,cis) Oktadekatrienoic Säure mit Triazetin in gegenwart eines Katalysators wie ein basiches Mittel, bei einer Temperatur und bei einer Druck wo das erhaltenes niedrig alkyl Azetate siedet, umsetzt, man die gewonnene Triglyzeride aus dem Gemisch von Mono-und Diglyzeride abtrennt, dann man mittels physikalischen Mitteln das Triglyzerid der cis,cis,cis Oktadekatrienoic Saüre (6,9,12) und das Diglyzerid von O-acetyl cis,cis,cis Oktadekatrienoic Saüre fraktionniert.

2. Verfahren nach Anspruch 1° worin das basiches Mittel ein Alkali Metal Alkoholate ist.

3. Verfahren nach einer der Ansprüche 1° und 2° worin das Alkalimetal Alkoholate Sodium Methanolate ist.

4. Verfahren nach einer der Ansprüche 1° und 2° worin das niedrig alkyl Ester von (cis,cis,cis) Oktadekatrienoic Saüre ein Ester mit 1 bis 4 Kohlenstoff Atomen ist.

5. Verfahren nach einer der Ansprüche 1° bis 4° worin die Trennung der Triglyzeriden mittels chromatographie auf Silica Saüle durchgeführt wird.

6. Verfahren nach einer der Anspruche 1° bis 5° worin das Gemisch von Triglyzeriden mittels High Performance Liquid Chromatography in inverse phase fraktionniert wird.

7. Verwendung von Triglyzerid der (cis,cis,cis) Oktadekatrienoic Saüre (6,9,12) in diatetischen Zubereitungen, sofern dieses nach der Verfahren der Anspruch 1 hergestellt ist.
